# EUROPÄISCHE PATENTSCHRIFT

(11) **EP 2 322 095 B1**
(45) Veröffentlichungstag und Bekanntmachung des Hinweises auf die Patenterteilung: **04.07.2018**
(21) Anmeldenummer: 09176217.9
(22) Anmeldetag: 17.11.2009
(51) Int. Cl.: A61B 5/151

(54) **Messgerät für Blutwerte**
Measuring device for blood parameters
Appareil de mesure pour valeurs sanguines

(43) Veröffentlichungstag der Anmeldung: 18.05.2011
(73) Patentinhaber: Beurer GmbH, 89077 Ulm (DE)
(72) Erfinder: Ernst Merk, 89264 Weissenhorn (DE); Werner Meternek, 89233 Neu -Ulm (DE); Thomas Strobel, 86480 Waltenhausen (DE); Marco Bühler, 89075 Ulm (DE)
(74) Vertreter: Fleck, Hermann-Josef

(56) Entgegenhaltungen:
- US-A1- 2005 096 565
- US-A1- 2007 152 683
- US-A1- 2007 233 395

## Beschreibung

Die Erfindung bezieht sich auf ein Messgerät für Blutwerte, insbesondere zur Analyse des Blutzuckergehalts, mit einer Vorrichtung zum Gewinnen von Blut und einem tegehäuse untergebracht ist.

In der US 2007/0233395 A1 ist ein Messgerät unter anderem für Blutdruckwerte mit einem Messteil beschreiben, der einen Teststreifen und eine Anschlusseinheit zur Datenübertragung aufweist. Bei einer alternativen Ausgestaltung ist auch eine Integration mit einem Stechteil allgemein angegeben.

Die US 2005/0096565 A1 zeigt ebenfalls ein Messgerät für Blutwerte mit einem Stechteil.

Ein weiteres Messgerät dieser Art ist in der CA 2 600 226 A1 angegeben. Auch dieses bekannte Messgerät ist als Handgerät ausgebildet und weist eine Aufnahme für einen Teststreifen zur Blutanalyse auf, um den Blutzuckergehalt zu bestimmen. Zum Anzeigen der Messwerte ist in dem Gerätegehäuse ein Anzeigefeld integriert, das auch zur visuellen Benutzerführung ausgestaltet ist und an eine Steuereinrichtung und Speichereinrichtung angeschlossen ist. Auf dem Bildschirm kann auch dargestellt werden, wie eine separate Vorrichtung zum Gewinnen eines Bluttropfens und dessen Aufgabe auf den Teststreifen zu handhaben ist. Dieses Messgerät bietet eine vorteilhafte Benutzerführung. Die Handhabung bei den Messvorgängen erfordert verschiedene Tätigkeiten von der Gewinnung der Körperflüssigkeit bis hin zur Überwachung der Messergebnisse und Beurteilung des Zustandes eines Patienten anhand der gewonnenen Messwerte und ist mit entsprechendem Aufwand verbunden.

Die WO2006/082174 A1 zeigt eine elektromechanische Stechhilfe zur Gewinnung flüssiger Proben, insbesondere von Bluttropfen zum Bestimmen des Blutzuckers. Bei diesem Gerät geht es nicht um die Analyse der Körperflüssigkeit an sich.

In der DE 103 32 488 A1 ist ein Analysegerät und Analyseverfahren für Blut mit integrierter Stecheinheit und einem Testband zum Untersuchen des gewonnenen Bluts gezeigt. Hierbei sind keine Angaben zu einer Datenverarbeitung und Darbietung zur Information des Benutzers gemacht. Ein ähnliches Messgerät für Blut, insbesondere Blutzucker, ist auch in der DE 198 49 539 A1 gezeigt.

Der Erfindung liegt die Aufgabe zugrunde, ein Messgerät für Blutwerte der eingangs genannten Art bereit zu stellen, das die Bedienfunktionen von der Gewinnung des Blutes bis zur Beurteilung durch eine prüfende Person möglichst benutzerfreundlich erfüllt.

Diese Aufgabe wird mit den Merkmalen des Anspruches 1 gelöst. Hierbei ist vorgesehen, dass die Vorrichtung zum Gewinnen des Blutes und die Analyseeinrichtung in einer kompakten, in einer Hand tragbaren Einheit integriert sind, die mit (mindestens oder nur) einer Anschlusseinheit zur Datenübertragung an ein separates Wiedergabegerät versehen ist.

Mit diesen Maßnahmen ergibt sich für den Benutzer eine einfache Handhabung des Messgerätes von der Gewinnung des Blutes, bis zur Kontrolle der Messwerte durch ihn oder z. B. einen Arzt. Durch die Datenübertragung wird auf einfache Weise auch
eine Überwachung der Messwerte über längere Zeiträume und/oder verschiedener Benutzer bzw. Patienten ermöglicht. So kann eine Analyse des Blutzuckergehalts oder anderer Stoffe wie Cholesterin (Totalwert, LDL und/oder HDL), Triglyceride, Laktat, Uric-Acid, HbA1c bei einfacher Bedienung vorgenommen werden.

Zu einer bedienungsfreundlichen Handhabung tragen die Maßnahmen bei, dass die Anschlusseinheit als ein mit dem Gerätegehäuse und/oder einer darin enthaltenen Platine starr, federnd oder klappbar verbundenes Steckteil ausgebildet ist, mit dem zumindest der Messteil des Messgerätes an dem separaten Auswertegerät ansteckbar und elektrisch zur Datenübertragung kontaktierbar ist. Auch trägt diese Maßnahme zu einer schlanken, gut handhabbaren Bauform bei.

Eine für die Datenübertragung und Datenverarbeitung vorteilhafte Ausgestaltung besteht darin, dass die Anschlusseinheit zur Verbindung mit einer standardisierten Schnittstelle, beispielsweise einer USB-Schnittstelle, des separaten Auswertegerätes ausgebildet ist.

Eine weitere vorteilhafte Ausgestaltung besteht darin, dass die Anschlusseinheit mit einem Übertragungsteil zur drahtlosen Übertragung, wie IR-Übertragung, Ultraschall-Übertragung oder Funkübertragung ausgestattet ist.

Eine für den Aufbau und die Handhabung vorteilhafte Ausgestaltung besteht ferner darin, dass das Gerätegehäuse aus zwei lösbar miteinander verbundenen Gehäuseabschnitten zusammengesetzt ist, wobei der erste Gehäuseabschnitt den Messteil und der zweite Gehäuseabschnitt die Vorrichtung zum Gewinnen des Blutes umfasst. Dabei brauchen die Gehäuseabschnitte nicht vollständig voneinander entfernbar zu sein, sondern können unverlierbar über ein Scharnier klappbar, über einen

Verschiebemechanismus verschiebbar oder über eine Schnur, ein Band, eine Kette oder dgl. flexibel aneinander gekoppelt bleiben, oder nach Trennen von der anderen Seite (nach dem Prinzip einer Füllfederhalterkappe) aufeinander steckbar ausgebildet sein.

Eine besondere vorteilhafte Ausführung besteht darin, dass die Vorrichtung zum Gewinnen des Bluts als Steckteil mit einer ein Stechmittel aufweisenden Stecheinheit ausgebildet ist.

Der Aufbau und die Funktion und Bedienung werden dadurch begünstigt, dass der Steckteil im Trennbereich zwischen den beiden Gehäuseabschnitten an dem ersten Gehäuseabschnitt mit dem Messteil angebracht ist und im verbundenen Zustand der beiden Gehäuseabschnitte in einen Hohlraum des zweiten Gehäuseabschnittes ragt. Dabei kann im Bereich des Hohlraumes eine Öffnung für ein Übertragungsglied zur drahtlosen Datenübertragung angeordnet sein.

Weitere vorteilhafte Maßnahmen für den Aufbau und die Bedienung bestehen darin, dass der Stechteil ein in dem zweiten Gehäuseabschnitt gelagertes, mit der Stecheinheit gekoppeltes Betätigungselement zum Einstellen der Einstechtiefe und/oder Auslösen der Stechfunktion aufweist.

Zu einem bedienungsfreundlichen Aufbau tragen ferner die Maßnahmen bei, dass in dem Gerätegehäuse, insbesondere in dem ersten Gehäuseabschnitt, eine Anzeigevorrichtung mit einem optischen Anzeigefeld für eine visuelle Anzeige und/oder eine Testmittelaufnahme integriert ist/sind.

Für die Funktion und den Einsatz des Messgerätes sind auch die Merkmale von Vorteil, dass der Messteil eine Speichervorrichtung aufweist, in der zumindest mittels der Analyseeinrichtung gewonnene Messdaten gespeichert werden. Des Weiteren ist die Speicherung zusätzlicher Daten, wie Kalibrierdaten, personenbezogener Daten, Bedienanweisungsdaten und dgl. möglich. Die Speichervorrichtung kann dabei in verschiedener Weise ausgebildet sein, beispielsweise als interner und/oder externer Speicher, als dynamischer, statischer und/oder flüchtiger Speicher, mit oder ohne Stromversorgung (Batterie, Akku) oder zusammengesetzt aus mehreren gleichen oder verschiedenen solcher Speichereinheiten.

Die Bedienung wird ferner dadurch begünstigt, dass bei Anschluss des Messgerätes mittels der Anschlusseinheit an das separate Wiedergabegerät die Übertragung der Messdaten automatisch erfolgt, wobei auch die Anzeige automatisch erfolgen kann. Die automatische Datenübertragung kann dabei unmittelbar durch den Einsteckvorgang bei vollständig eingesteckter Anschlusseinheit oder nach Freigabe durch den Benutzer ausgelöst werden. Dabei kann eine sogenannte HID-Technik (Human Interface Device) zur automatischen Erkennung des angeschlossenen Messgerätes eingesetzt werden mit einem Universalgerätetreiber, der das Messgerät erkennt, wobei ein Boot-Vorgang durchgeführt wird. Auch ist eine manuelle Auslösung der Übertragung durch den Benutzer möglich.

Zur Benutzerführung tragen die Maßnahmen bei, dass in der Speichervorrichtung Zusatzdaten, insbesondere personenbezogene Daten und/oder zeitbezogene Daten, hinterlegt oder hinterlegbar sind, und dass in dem Messteil eine Datenverwaltungseinrichtung vorhanden ist, mittels derer den Messdaten betreffende Zusatzdaten zuordenbar sind.

Mit den Maßnahmen, dass das Messgerät zum Anschluss an eine externe Speichervorrichtung ausgebildet ist, wird ein benutzerfreundlicher Aufbau unterstützt.

Eine für die Datensicherheit vorteilhafte Ausbildung besteht darin, dass das Messteil mit einer Einrichtung zur codierten Datenübertragung, insbesondere einer Datenverschlüsselungseinrichtung, versehen ist.

Die Erfindung wird nachfolgend anhand von Ausführungsbeispielen unter Bezugnahme auf die Zeichnungen näher erläutert. Es zeigen:
- Fig. 1A und 1B: ein Ausführungsbeispiel eines Messgerätes für Blutwerte in einer perspektivischen Ansicht von schräg vorn oben von einer Stecheinheit aus und von schräg hinten oben von einer Testmittelaufnahme aus,
- Fig. 1C: einen Messteil des Messgerätes nach Fig. 1A von einem Trennbereich aus,
- Fig. 1D: einen Stechteil des Messgerätes nach Fig. 1A von einem Trennbereich aus,
- Fig. 2A und 2B: ein weiteres Ausführungsbeispiel des Messgerätes in perspektivischer Ansicht von schräg vorn oben von einer Stecheinheit aus und von schräg hinten oben von einer Testmittelaufnahme aus,
- Fig. 2C: den Messteil des Messgerätes nach Fig. 2A in perspektivischer Ansicht von einem Trennbereich aus,
- Fig. 2D: den Stechteil des Messgerätes nach Fig. 2A in perspektivischer Ansicht von dem Trennbereich aus,
- Fig. 3: eine perspektivische Teilansicht des Messgerätes nach Fig. 1A bei einem abgenommenen unteren Gehäuseteil in perspektivischer Ansicht von schräg oben,
- Fig. 4: die Teilansicht des Messgerätes nach Fig. 3 in umgedrehter Darstellung in das Innere des Messgerätes in perspektivischer Ansicht,
- Fig. 5: Einbaukomponenten des Messgerätes nach Fig. 1 im Bereich des Messteils ohne Gehäuse und
- Fig. 6: eine Blockdarstellung zu dem Messgerät mit elektronischen Schaltungskomponenten.

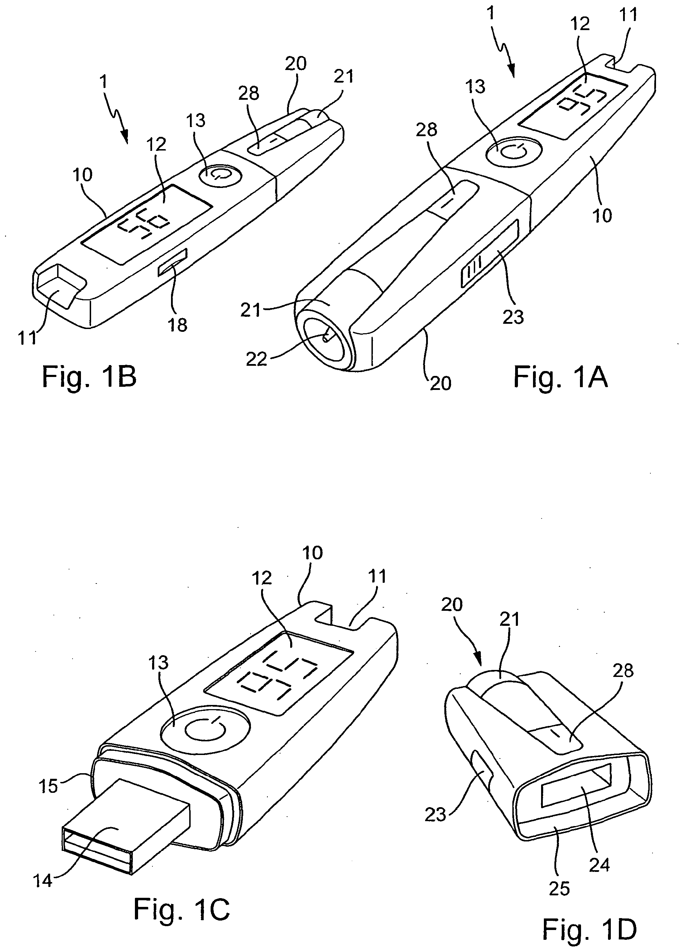

Fig. 1A zeigt ein erstes Ausführungsbeispiel eines Messgeräts zur Analyse und Messung von Blutwerten, vorliegend des Zuckergehaltes im Blut. Das Messgerät 1 besitzt ein lang gestrecktes Gerätegehäuse, das leicht in einer Hand gehalten werden kann, wobei es mit seiner Unterseite auf der Handinnenfläche liegt und seine Oberseite mit einem optischen Anzeigefeld 12 zur visuellen Informationsdarbietung dem Benutzer zugewandt ist, während die Finger eine Seitenfläche umgreifen und der Daumen entlang der gegenüber liegenden Seitenfläche sich erstreckt oder diese umgreift.

Das Messgerät 1 setzt sich aus einem Messteil 10 in einem ersten Teilgehäuse bzw. ersten Gehäuseabschnitt und einem Stechteil 20 in einem zweiten Teilgehäuse bzw. zweiten Gehäuseabschnitt zusammen. Das Anzeigefeld 12 sowie mindestens ein Bedienelement 13 sind in dem ersten Teilgehäuse aufgenommen. Das zweite Teilgehäuse nimmt eine Stecheinheit 21 mit einem Stechmittel 22, wie Stechlanzette oder dgl. auf, und ist mit einem oder mehreren Betätigungselementen, wie einem Spannteil 23 und einem Auslöser 28 zum manuellen Einwirken auf die Stecheinheit 21 mit dem Stechmittel 22 versehen. Das Stechmittel 22 ist in seiner Längserstreckung parallel zur Längserstreckung des Messgerätes in der Stecheinheit 21 gelagert und liegt mit seinem freien Ende zentral in einem auf der einen, vorderen Schmalseite des Gerätegehäuses, nämlich des zweiten Teilgehäuses mittig angeordneten vertieften Bereich. Auf der anderen, hinteren Schmalseite des Gerätegehäuses, nämlich des ersten Teilgehäuses, ist eine Testmittelaufnahme 11 für ein Testmittel zur Analyse des Blutes, insbesondere also eines Bluttropfens ausgebildet. In Fig. 1B, das das Messgerät nach Fig. 1A von der hinteren Schmalseite aus zeigt, ist die Testmittelaufnahme 11 genauer zu sehen. Ein als Teststreifen ausgeführtes Testmittel 30 kann in einen Einführschlitz der Testmittelaufnahme 11 eingeführt und im Inneren des Messteils 10 analysiert werden, wobei die Messwerte in dem Messteil 10 verarbeitet und über das Anzeigefeld 12 zur Anzeige gebracht werden. Der Einführschlitz ist in einer vertieften Ausnehmung an der hinteren Schmalseite angeordnet, die nach oben offen und beidseitig sowie unten von einem vorstehenden Rand umgeben ist, wobei die Schlitzöffnung für den Teststreifen in Verlängerung der unteren Begrenzungsfläche der Ausnehmung angeordnet ist. Mit dieser Ausbildung lässt sich der Teststreifen leicht in das Messgerät 1 einführen. Beispielsweise an dem Gehäuseabschnitt des Messteils 10 ist ein Betätigungsmittel 18, etwa ein Wipptaster, für weitere Einstellungen, wie z. B. eine Uhrzeitwahl für eine Analyse, angeordnet.

Wie die Fig. 1C und 1D weiter zeigen, sind der Messteil 10 und der Stechteil 20 in einem Trennbereich miteinander verbindbar, beispielsweise zusammensteckbar, ineinander klipsbar, verrastbar, verhakbar, klappbar, gegeneinander verschiebbar oder ineinander drehbar, wozu entsprechend ausgebildete Verbindungsmittel mit einem an dem Messteil 10 im Trennbereich angeordneten Verbindungsabschnitt 15 und mit einem an dem Stechteil 20 in dem Trennbereich angeordneten an den Verbindungsabschnitt 15 angepassten Verbindungsgegenabschnitt 25 vorhanden sind. In Längsrichtung des Messteils 10 steht aus diesem im Trennbereich eine Anschlusseinheit 14 vor, die starr mit dem Messteil 10 verbunden ist und an in dem Messteil 10 angeordnete elektronische Schaltungskomponenten angeschlossen ist. Vorliegend ist die Anschlusseinheit 14 als USB-Steckeinheit ausgebildet. In dem Stechteil 20 ist ein auf die Anschlusseinheit 14 abgestimmter Hohlraum 24 angeordnet, in den die Anschlusseinheit 14 im zusammengesetzten Zustand des Messteils 10 und des Stechteils 20 hineinragt, wobei das zweite Teilgehäuse eine Schutzkappe für die Anschlusseinheit 14 bildet. Weitere in den Fig. 1C und 1D gezeigte Teile entsprechen den mit gleichen Bezugszeichen versehenen Teilen nach Fig. 1A und 1B. Wie insbesondere aus Fig. 1C ersichtlich, ist das Bedienelement 13 in einer geringen Vertiefung angeordnet, so dass ein gewisser Auslöseschutz und zudem eine gute taktile Erkennung gegeben sind.

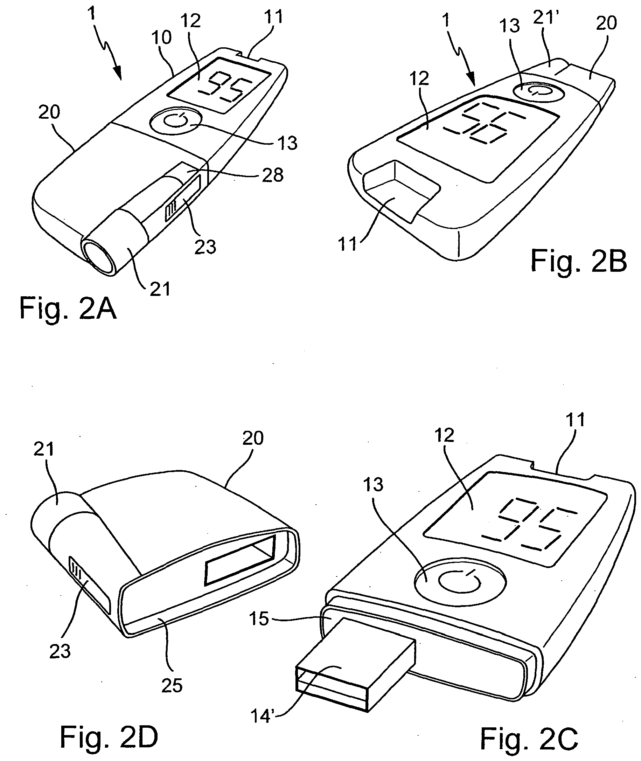

Die Fig. 2A und 2B zeigen ein weiteres Ausführungsbeispiel des Messgerätes 1, das sich ebenfalls aus einem Messteil 10 und einem Stechteil 20 zusammensetzt, die in einem Trennbereich miteinander lösbar verbunden sind. Bei diesem Ausführungsbeispiel ist die Stecheinheit 21 im Unterschied zu der Ausführung nach den Fig. 1A bis 1D nicht zentral in der vorderen Schmalseite angeordnet, sondern seitlich und die Form des Gerätegehäuses ist gegenüber dem ersten Ausführungsbeispiel verkürzt. Dies gelingt dadurch, dass, wie aus den Fig. 2C und 2D ersichtlich, die Anschlusseinheit 14' seitlich von der Mittellängsachse zu der von der Stecheinheit 21 abgelegenen Seite hin versetzt ist. Die parallel zur Längsachse des Messgerätes 1 vorstehende Anschlusseinheit 14' ragt im zusammengesetzten Zustand der Teilgehäuse ebenfalls in einen Hohlraum 24 des Stechteils 20, wozu der Hohlraum entsprechend der Lage der Anschlusseinheit 14' positioniert ist. Der Verbindungsabschnitt 15 des Messteils 10 im Trennbereich und der Verbindungsgegenabschnitt 25 des Stechteils 20 können entsprechend den vorstehend im Zusammenhang mit dem ersten Ausführungsbeispiel angegebenen Ausführungen gestaltet sein. Die Testmittelaufnahme 11 ist auch hierbei zur Aufnahme eines Teststreifens ausgeführt, wobei ein Einführschlitz entsprechend dem ersten Ausführungsbeispiel in einer Ausnehmung angeordnet ist.

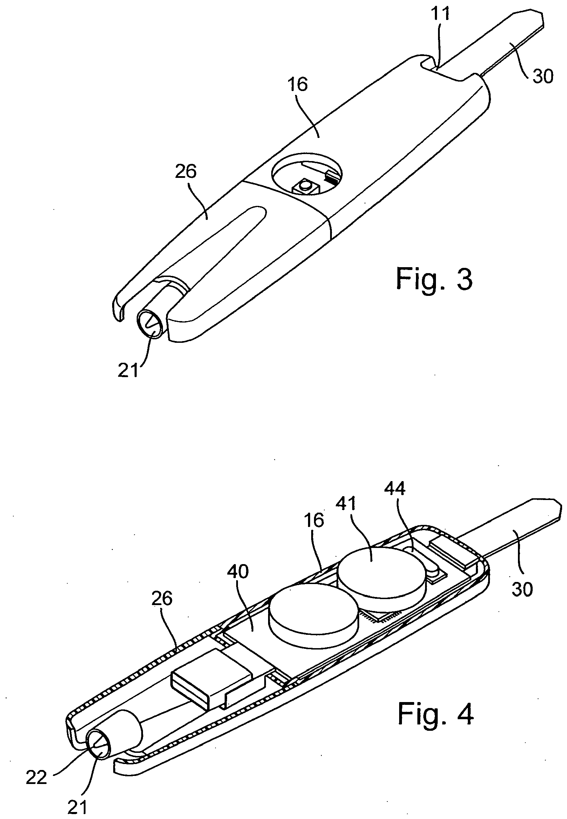

Fig. 3 zeigt eine Teilansicht des Messgerätes 1, wobei das dem Messteil 10 zugeordnete erste Teilgehäuse ein oberes Gehäuseteil 16 und das dem Stechteil 20 zugeordnete zweite Teilgehäuse ein weiteres oberes Gehäuseteil 26 aufweisen und mit diesen beiden Gehäuseteilen 16 und 26 verbindbare (nicht gezeigte) untere Gehäuseteile abgenommen sind. Auch ein Deckelbereich in dem oberen Gehäuseteil 26 des Stechteils 20 über dem vorderen Bereich der Stecheinheit 21 ist abgenommen. Durch eine Öffnung in dem oberen Gehäuseteil des Messteils 10, in dem das Bedienelement 13 anzuordnen ist, sind einige elektronische Bauteile ersichtlich. In der Testmittelaufnahme 11 ist ein Testmittel 30, vorliegend ein Teststreifen angeordnet.

Fig. 4 zeigt die Teilansicht nach Fig. 3 von der Unterseite, wobei eine Platine 40 mit elektrischen Komponenten des Messteils 10, wie elektrische Schaltungskomponenten 43, Schwingquarz 44, Batterie 41 und/oder Akku sowie die daran angeschlossene Anschlusseinheit 14 und auch ein Tasterbaustein 42 zu sehen sind.

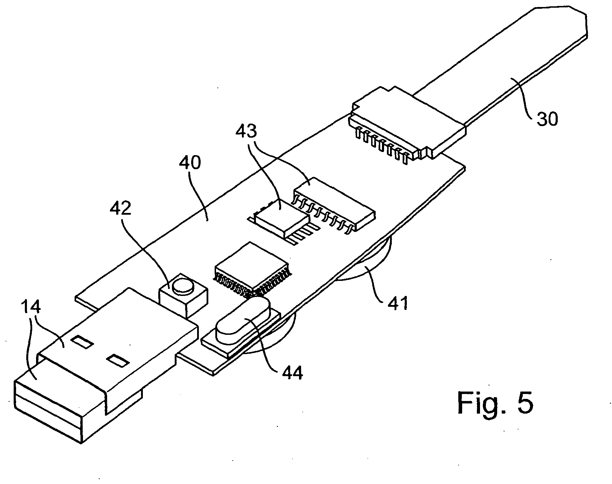

Fig. 5 zeigt die Platine 40 des Messteils 10 mit darauf angeordneten elektrischen Schaltungskomponenten sowie die daran angeschlossene Anschlusseinheit 14 bei entfernten Gehäuseteilen des Messteils 10. In einen Analysebaustein des Messteils 10 ist das Testmittel 30 eingesetzt.

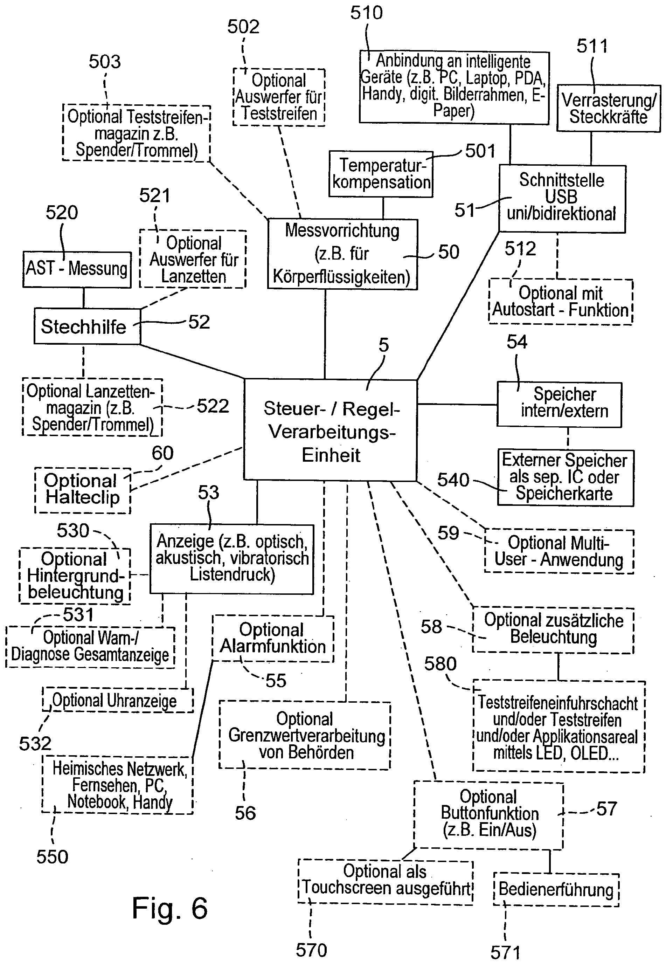

Fig. 6 zeigt in einem Blockschaltbild wesentliche Komponenten des Messgerätes 1. An eine Zentraleinheit 5, die Steuerungs-, Regelungs- und Datenverarbeitungsfunktionen erfüllt, sind eine Messvorrichtung 50, insbesondere für die Körperflüssigkeiten, eine Schnittstelle 51, beispielsweise eine uni- oder bidirektionale USB-Schnittstelle, eine Stechhilfe 52, eine Anzeige 53 mit optischen, akustischen oder vibratorischen Anzeigekomponenten oder mit Komponenten für einen Listendruck und eine interne und/oder externe Speichereinrichtung 54 in Signal- bzw. Datenübertragungsverbindung gebracht. Optional können des Weiteren Komponenten für eine Alarmfunktion 55, für eine Grenzwertverarbeitung 56, für Betätigungselementfunktionen 57, für zusätzliche Beleuchtungsfunktionen 58, für eine Mehrfachbenutzer-Anwendung 59 und/oder z. B. ein Halteclip 60 angeschlossen sein, so dass auch diesbezügliche Daten an die Zentraleinheit 5 übertragbar und von ihr verarbeitbar sind.

Eine vorteilhafte Ausgestaltung der Messvorrichtung 50 besteht darin, dass diese mit einer Temperaturkompensation 501 versehen ist. Ferner kann die Messvorrichtung 50 mit einem Auswerfer 502 für Teststreifen und/oder mit einem Teststreifenmagazin 503 z. B. mit Spender bzw. Trommel gekoppelt sein.

Die Schnittstelle 51 ist mit einer Anbindung 510, insbesondere der Anschlusseinheit 14, 14' versehen und darüber an ein separates Wiedergabegerät bzw. intelligentes Gerät, wie z. B. Personalcomputer, Tischcomputer, transportabler Computer, Taschencomputer, Handtelefon, digitaler Bilderrahmen und/oder E-Paper anschließbar.

Hierzu ist vorteilhaft eine Gerätekopplung 511 mit die Funktionssicherheit gewährleistenden Steckkräften bzw. einer Verrastung versehen. Dabei kann die Schnittstelle 51 mit einer Autostart-Funktion 512 ausgestattet sein, wodurch eine einfache Bedienung bei der Datenübertragung und Auswertung unterstützt wird.

Die Stechhilfe 52 ist vorteilhaft mit einer Vorrichtung zur AST-Messung (Alternate-Site-Testing) 520 bestückt oder bestückbar. Zudem kann sie mit einem Lanzettenauswerfer 521 für die Stechmittel 22 und/oder einem Lanzettenmagazin 522, wie Spender oder Trommel, versehen oder koppelbar sein.

Die Anzeigeeinrichtung 53 kann in verschiedener Weise ausgestaltet sein, z. B. mit einer Hintergrundbeleuchtung 530, einer Warn-Diagnose-Anzeige 531, die sich auf die Gesamtanzeige beziehen kann, z. B. rote Unterlegung oder Umschaltung in einen Blinkmodus oder dgl., und/oder mit einer Zeitanzeige (Uhr, Datum 532).

Die Speichereinrichtung 54 kann mit einer externen Speichereinheit 540 verbindbar ausgestaltet sein, wie z. B. einer separaten integrierten Schaltung oder einer Speicherkarte oder dgl.

Die Vorrichtung mit der Alarmfunktion 55 ist z. B. in der Weise ausgebildet, dass sie Warn- oder Alarmmeldungen über ein heimisches Netzwerk an einem Fernseher, einem Personalcomputer oder transportablen Computer, einem Handtelefon oder an anderen Netzwerkgeräten 550 ausgeben kann.

Die Betätigungsmittel 57 können eine berührungsempfindliche und/oder näherungsempfindliche Bildschirmfläche (resistiv oder kapazitiv oder induktiv) 570 und/oder eine einblendbare Bedienerführung 571, die sich entsprechend den verschiedenen erforderlichen Bedienanweisungen ändert, ausgerüstet sein.

Die Zusatzbeleuchtung 58 umfasst z. B. Untereinheiten 580 mit Beleuchtungsmitteln für die Testmittelzuführung, Messteile und/oder die Anschlusseinheit 14, 14', wie z. B. einen USB-Anschluss. Die Zusatzbeleuchtung ist vorteilhaft in die Benutzerführung durch entsprechende Ansteuerung, insbesondere Softwaresteuerung einbezogen. So können betreffende zu betätigende Stellen nacheinander angesteuert und ausgeleuchtet bzw. signalisiert werden, etwa kontinuierlich, blinkend oder farbcodiert, so dass die Bedienvorgänge erleichtert werden, insbesondere für auf Hilfe angewiesene Personen.

Alle an die Messvorrichtung 50 in Signalübertragungsverbindung gebrachten Einheiten, nämlich Schnittstelle 51, Stechhilfe 52, Anzeigeeinrichtung 53, Speichervorrichtung 54, Alarmfunktion 55, Grenzwertverarbeitungseinheit 56, Betätigungsmittel 57, Zusatzbeleuchtung 58, Mehrfachnutzer-Anwendung 59 und/oder Halteclip 60, die einzeln oder in Kombination aus mindestens zweien dieser Einheiten vorhanden sein können, sind für den Uni- und/oder, soweit zweckmäßig, für den bidirektionalen Signal- bzw. Datenaustausch mit der Zentraleinheit 5 ausgelegt, die die Steuerungs-, Regelungs- und/oder weitere Verarbeitungsaufgaben übernimmt und mit einer entsprechenden Datenverwaltungseinrichtung versehen ist. Ferner werden von der Zentraleinheit 5 aus und/oder den genannten Einheiten aus die jeweils daran angeschlossenen Untereinheiten (501, 502, 503; 510, 511, 512; 520, 521, 522; 530, 531, 532; 540; 550; 570, 571; 580) gesteuert oder geregelt. Mit diesen Maßnahmen wird ein leicht handhabbares, benutzerfreundlich ausgebildetes Messgerät für Körperflüssigkeiten, insbesondere Messgerät zur Analyse des Blutzuckergehalts realisiert, das der Benutzer wegen seiner geringen Größe und seinem geringen Gewicht und seiner ergonomisch günstigen Form leicht mit sich führen und handhaben kann. Gleichzeitig werden einfache Anschlussmöglichkeiten an separaten Wiedergabegeräten gegebenenfalls mit weiteren Verarbeitungsmöglichkeiten geboten.

Der Messteil 10 und der Stechteil 20 können mechanisch mittels eines Bandes, Kunststoffklipses, einer Gliederkette oder dgl. miteinander verbunden sein, womit einem Verlust vorgebeugt wird. In der Speichereinrichtung 54 gespeicherte Daten umfassen die aus der Analyse gewonnenen Messdaten und vorteilhaft Zusatzdaten zur Person des Benutzers, zu Aufnahmezeiten und/oder Darstellungsformen wie Einblendung von Warnhinweisen, Angabe von Schwellenwerten bzw. von Grenzwerten, die z. B. von Behörden vorgeschrieben sind, graphische Darstellungsformen, visuelle Darbietung und gleichzeitig akustische Signalgabe oder dgl.

Die Anzeigeeinrichtung 53 kann neben einer flächigen variablen Anzeige z. B. auf LCD-Basis, Punkt-Matrix-Basis oder dgl. einzelne Zusatzelemente wie verschiedenfarbige Leuchtdioden umfassen. Ferner können den übertragenen Daten Warnmeldungen zugeordnet werden, die dann auf dem separaten Wiedergabegerät zu betreffenden Warnhinweisen bzw. Alarmen führen.

Die gespeicherten Zusatzdaten können auch Programme z. B. zur Überprüfung des Batteriezustandes, unzulässig langer Messzeiträume, zur Diagnose z. B. von häufigen Fehlmessungen oder fehlerhaften Gerätefunktionen, für Prüfmittel zum Überwachen von Teststreifen und zum Hinweis auf unzulässige Teststreifen oder eine falsche Messstreifentemperatur und dgl. beinhalten und auch z. B. für Aufforderungen zu Messwiederholungen bei unsicheren Ergebnissen. Weitere Ausgestaltungsvarianten bestehen darin, dass automatisch Meldungen an einen Arzt, Krankendienst oder andere Pflege- oder Rettungseinrichtungen z. B. über Netzwerke kabelgebunden oder kabellos übermittelt werden.

Das Gerätegehäuse ist aus einem griffigen Material, z. B. unbeschichtetem oder beschichtetem Kunststoff oder Metall mit guter Haptik ausgebildet, wobei insbesondere auch die Trennstellen möglichst verschleißfrei ausgeführt sind. Ist die Verbindung zwischen Messteil 10 und Stechteil 20 als Drehverbindung ausgeführt, sind der Verbindungsabschnitt 15 und der Verbindungsgegenabschnitt 25 im Querschnitt kreisförmig ausgeführt und z. B. mit Gewinde und Gegengewinde oder als Bajonettverschluss mit betreffenden komplementären Eingriffelementen ausgestaltet.

Die Stechhilfe 52 an dem Stechteil 20 mit der Stecheinheit 21 und dem Stechmittel 22 funktioniert z. B. in der Weise, dass eine zum Schutz der Stechhilfe evtl. vorhandene Gerätekappe aufgedreht bzw. abgezogen wird, eine Nadel bzw. Lanzette eingelegt oder eingesteckt wird, eine Schutzscheibe von der Nadel bzw. Lanzette abgedreht wird, die Kappe wieder aufgedreht bzw. aufgedrückt wird, die Stecheinheit 21 mit dem Stechmittel 22 zur Vorgabe der Tiefe des Einstiches eingestellt wird, eine Federvorspannung erzeugt wird und zum Stechen ein Auslöser 28 betätigt wird, z. B. durch manuelle oder automatisch gesteuerte Einwirkung auf eine Entriegelung. Die sterile Nadel bzw. Lanzette steckt in einem Lanzettenhalter aus Metall, Kunststoff oder dgl. Dieser kann Teil einer Führung sein und bringt auch die Haltekraft für die Federvorspannung auf. Der Auslösevorgang kann mittels eines mechanischen Auslösers 28 oder Magnetauslösers oder anderer von der Zentraleinheit 5 und/oder der Stechhilfe 52 gesteuerter Aktoren erfolgen, wonach das gewonnene Analyt, z. B. der Bluttropfen auf das Testmittel, beispielsweise einen Teststreifen aufgetragen und die Messung ausgelöst wird, die Nadel bzw. Lanzette abgezogen wird und eine stechsichere Entsorgung erfolgt und die Gerätekappe wieder aufgesetzt wird. Mittels der Testmittelmethode können weitere aus dem Blut gewonnene Signale erfasst, ausgewertet, angezeigt, gespeichert und noch weiter verarbeitet werden. In der Stechhilfe 52 ist beispielsweise ein Magazin integriert, zur Aufnahme von Nadeln bzw. Lanzetten und/oder Teststreifen.

Die Anzeigeeinrichtung 53 umfasst das Anzeigefeld 12 mit z. B. LCD-Anzeige oder einer Punktmatrixanzeige, OLED, E-Paper mit oder ohne Eigenbeleuchtung. Die visuelle Informationsdarbietung kann dabei mit einem Lagesensor und einer Richtungssteuerung gekoppelt sein, so dass der Betrachtungswinkel für den Benutzer stets optimal eingehalten wird. Eine Beleuchtung an der Testmittelaufnahme 11 ist vorteilhaft mit einem Blinkmodus ausgestattet, der bei unzureichender Blutmenge für die Glucosemessung blinkt und bei ausreichend Blut auf Dauerlichtanzeige umschaltet bzw. von einer Farbe (z. B. rot oder gelb) auf eine andere (z. B. grün) umschaltet. Nach einer gewissen Zeit von einigen Sekunden, z. B. 5 Sekunden, kann an der Testmittelaufnahme 11 ein Entnahmeleuchtsignal eingeschaltet werden, nach einigen weiteren Sekunden, z. B. insgesamt 10 Sekunden, kann eine Abschaltung der Beleuchtung an der Testmittelaufnahme 11 und gegebenenfalls auch auf dem Anzeigefeld 12 erfolgen. In weiterer Ausgestaltung kann auch eine Tastenabfrage gespeicherter Messwerte mit oder ohne Beleuchtung bei Tastenabfrage erfolgen. Entsprechende Abfrage- und Einstellmöglichkeiten sind vorteilhaft auch für die Uhr-/ bzw. Datumsfunktion vorhanden. Die Anzeige kann auch mit auffälliger Blink- bzw. Blitzlichtabgabe ausgerüstet sein, gleichzeitig eine Taschenlampenfunktion erfüllen und/oder zur Hilfeaktivierung gegebenenfalls in Verbindung mit einer akustischen Anzeige oder durch eine akustische Anzeige allein ausgebildet sein, so dass schnell Hilfe bei einer Unterzuckerung, einem Schwächeanfall bzw. einer anderen Notfallsituation herbeigeholt werden kann.

Vorteilhaft ist die Steuerung bzw. Regelung des Messgerätes mit einem Mikrocontroller, insbesondere zum Erfüllen der Funktionen der Zentraleinheit 5 und gegebenenfalls weiterer Funktionen ausgestattet, so dass die Funktionsabläufe zumindest großenteils mittels Software-Programmen bereitgestellt und gegebenenfalls geändert oder ergänzt werden können. Hierbei kann auch der Datensicherheit vorteilhaft durch eine Datenverschlüsselungseinrichtung zur gesicherten Datenübertragung Rechnung getragen werden. Sensible Daten können dadurch z. B. nur für autorisierte Personen auf das Wiedergabegerät übertragen und/oder dort speicherbar bzw. sichtbar gemacht werden. Die Datenverschlüsselungseinrichtung kann z. B. mit einer programmierbaren Datenfiltervorrichtung ausgestattet sein.

## Patentansprüche

1. Messgerät (1) für Blutwerte, insbesondere zur Analyse des Blutzuckergehalts, mit einer Vorrichtung zum Gewinnen von Blut und einem eine Analyseeinrichtung für dasselbe umfassenden Messteil (10), das in einem Gerätegehäuse untergebracht ist, wobei die Vorrichtung zum Gewinnen des Blutes und die Analyseeinrichtung in einer kompakten, in einer Hand tragbaren Einheit integriert sind, die mit einer Anschlusseinheit (14, 14') zur Datenübertragung an ein separates Wiedergabegerät versehen ist, wobei das Gerätegehäuse aus zwei lösbar miteinander verbundenen Gehäuseabschnitten zusammengesetzt ist, von denen der erste Gehäuseabschnitt den Messteil (10) und der zweite Gehäuseabschnitt die Vorrichtung zum Gewinnen des Blutes umfasst, und wobei die Vorrichtung zum Gewinnen des Blutes als Stechteil (20) mit einer ein Stechmittel (22) aufweisenden Stecheinheit (21) ausgebildet ist,
**dadurch gekennzeichnet,**
**dass** die Anschlusseinheit (14, 14') als ein mit dem Gerätegehäuse und/oder einer darin enthaltenen Platine (40) starr, federnd oder klappbar verbundenes Steckteil ausgebildet ist, mit dem zumindest der Messteil (10) des Messgerätes (1) an dem separaten Wiedergabegerät ansteckbar und elektrisch zur Datenübertragung kontaktierbar ist,
**dass** der Steckteil im Trennbereich zwischen den beiden Gehäuseabschnitten an dem ersten Gehäuseabschnitt mit dem Messteil (10) angebracht ist und im verbundenen Zustand der beiden Gehäuseabschnitte in einen auf die Anschlusseinheit (14, 14') abgestimmten Hohlraum (24) des zweiten Gehäuseabschnittes ragt, wobei Verbindungsmittel mit einem an dem Messteil (10) im Trennbereich angeordneten Verbindungsabschnitt (15) und mit einem an dem Stechteil (20) in dem Trennbereich angeordneten, an den Verbindungsabschnitt (15) angepassten Verbindungsgegenabschnitt (25) vorhanden sind, und der zweite Gehäuseabschnitt eine Schutzkappe für die Anschlusseinheit (14, 14') bildet und dass der Stechteil (20) ein in dem zweiten Gehäuseabschnitt gelagertes, mit der Stecheinheit (21) gekoppeltes Betätigungselement zum Einstellen der Einstechtiefe und/oder Auslösen der Stechfunktion aufweist.

2. Messgerät nach Anspruch 1,
**dadurch gekennzeichnet,**
**dass** die Anschlusseinheit (14, 14') zur Verbindung mit einer standardisierten Schnittstelle, beispielsweise einer USB-Schnittstelle, des separaten Wiedergabegerätes ausgebildet ist.

3. Messgerät nach einem der vorhergehenden Ansprüche
**dadurch gekennzeichnet,**
**dass** die Anschlusseinheit (14, 14') mit einem Übertragungsteil zur drahtlosen Datenübertragung, wie IR-Übertragung, Ultraschall-Übertragung oder Funkübertragung, ausgestattet ist.

4. Messgerät nach einem der vorhergehenden Ansprüche,
**dadurch gekennzeichnet,**
**dass** in dem Gerätegehäuse, insbesondere in dem ersten Gehäuseabschnitt, eine Anzeigevorrichtung mit einem optischen Anzeigefeld und/oder eine Testmittelaufnahme (11) integriert ist/sind.

5. Messgerät nach einem der vorhergehenden Ansprüche,
**dadurch gekennzeichnet,**
**dass** der Messteil (10) eine Speichervorrichtung aufweist, in der mittels der Analyseeinrichtung gewonnene Messdaten gespeichert werden.

6. Messgerät nach Anspruch 5,
**dadurch gekennzeichnet,**
**dass** bei Anschluss des Messgerätes (1) mittels der Anschlusseinheit (14, 14') an das separate Wiedergabegerät die Übertragung der Messdaten automatisch erfolgt.

7. Messgerät nach Anspruch 5 oder 6,
**dadurch gekennzeichnet,**
**dass** in der Speichervorrichtung Zusatzdaten, insbesondere personenbezogene Daten und/oder zeitbezogene Daten, hinterlegt oder hinterlegbar sind, und dass in dem Messteil (10) eine Datenverwaltungseinrichtung vorhanden ist, mittels derer den Messdaten betreffende Zusatzdaten zuordenbar sind.

8. Messgerät nach einem der vorhergehenden Ansprüche,
**dadurch gekennzeichnet,**
**dass** das Messgerät (1) zum Anschluss an eine externe Speichervorrichtung ausgebildet ist.

9. Messgerät nach einem der vorhergehenden Ansprüche,
**dadurch gekennzeichnet,**
**dass** das Messteil (10) mit einer Einrichtung zur codierten Datenübertragung, insbesondere einer Datenverschlüsselungseinrichtung, versehen ist.

## Claims

1. Measuring instrument (1) for blood values, in particular for analyzing blood sugar content, which comprises a device for extracting blood and a measuring part (10) comprising an analysis apparatus for said blood, and which is accommodated in an instrument housing, the device for extracting the blood and the analysis apparatus being integrated in a compact handheld unit that is provided with a connection unit (14, 14') for transmitting data to a separate reproduction device, the instrument housing being composed of two detachably interconnected housing portions, the first housing portion of which comprises the measuring part (10) and the second housing portion of which comprises the device for extracting the blood, and the device for extracting the blood being designed as a piercing part (20) having a piercing unit (21) that comprises a piercing means (22), **characterized in that** the connection unit (14, 14') is designed as a plug-in part which is rigidly, resiliently or hingedly connected to the instrument housing and/or to a circuit board (40) contained therein, and by means of which at least the measuring part (10) of the measuring instrument (1) can be plugged onto the separate reproduction device and brought into electrical contact therewith in order to transmit data, **in that** the plug-in part is attached to the first housing portion comprising the measuring part (10) in the separating region between the two housing portions and, when the two housing portions are connected, projects into a cavity (24) in the second housing portion, which cavity is adapted to the connection unit (14, 14'), connection means being provided that comprise a connection portion (15) that is arranged on the measuring part (10) in the separating region and a counter connection portion (25) that is arranged on the piercing part (20) in the separating region and is adapted to the connection portion (15), and the second housing portion forming a protective cap for the connection unit (14, 14'), and **in that** the piercing part (20) comprises an actuation element which is mounted in the second housing portion, is coupled to the piercing unit (21), and is intended for adjusting the piercing depth and/or for triggering the piercing function.

2. Measuring instrument according to claim 1, **characterized in that** the connection unit (14, 14') is designed for connection to a standardized interface of the separate reproduction device, for example a USB interface thereof.

3. Measuring instrument according to either of the preceding claims, **characterized in that** the connection unit (14, 14') is equipped with a transmission part for wireless data transmission, for example infrared transmission, ultrasonic transmission or radio transmission.

4. Measuring instrument according to any of the preceding claims, **characterized in that** a display device comprising an optical display panel and/or a test means holder (11) is/are integrated in the instrument housing, in particular in the first housing portion.

5. Measuring instrument according to any of the preceding claims, **characterized in that** the measuring part (10) comprises a storage device in which measurement data obtained by means of the analysis apparatus are stored.

6. Measuring instrument according to claim 5, **characterized in that** the measurement data are automatically transmitted when the measuring instrument (1) is connected to the separate reproduction device by means of the connection unit (14, 14').

7. Measuring instrument according to either claim 5 or claim 6, **characterized in that** additional data, in particular personal data and/or time-related data, are or can be stored in the storage device, and **in that** a data management apparatus is provided in the measuring part (10), by means of which apparatus relevant additional data can be assigned to the measurement data.

8. Measuring instrument according to any of the preceding claims, **characterized in that** the measuring instrument (1) is designed for connection to an external storage device.

9. Measuring instrument according to any of the preceding claims, **characterized in that** the measuring part (10) is provided with an apparatus for transmitting data in an encoded manner, in particular a data encryption apparatus.

## Revendications

1. Appareil de mesure (1) pour valeurs sanguines, en particulier pour l'analyse du niveau de glycémie, comprenant un dispositif de prélèvement de sang et une partie mesure (10) qui est pourvu d'un dispositif d'analyse de sang et qui est logé dans un boîtier d'appareil, le dispositif de prélèvement du sang et le dispositif d'analyse étant intégrés dans une unité compacte qui peut être tenue d'une main et qui est dotée d'une unité de raccordement (14, 14') destinée à transmettre des données à un appareil de lecture séparé,
le boîtier d'appareil étant composé de deux parties de boîtier reliées l'une à l'autre de manière amovible et dont la première partie de boîtier comprend la partie mesure (10) et la seconde partie de boîtier comprend le dispositif de prélèvement du sang, et le dispositif de prélèvement du sang étant conçu comme une partie piqûre (20) dotée d'une unité de piqûre (21) comportant un moyen de piqûre (22),
**caractérisé en ce que**
l'unité de raccordement (14, 14') est conçue comme un connecteur mâle lié de manière fixe, élastique ou rabattable au boîtier d'appareil et/ou à un circuit imprimé (40) présent à l'intérieur et au moyen duquel au moins la partie mesure (10) de l'appareil de mesure (1) peut être enfichée dans un appareil de lecture séparé et mise en contact électrique avec celui-ci pour la transmission de données,
le connecteur mâle est monté sur la première partie de boîtier, comportant la partie mesure (10), dans la zone de séparation située entre les deux parties de boîtier et fait saillie dans une cavité (24) de la seconde partie de boîtier qui est adaptée à l'unité de raccordement (14, 14') lorsque les deux parties de boîtier sont reliées, des moyens de liaison étant prévus lesquels comportent une section de liaison (15) disposée au niveau de la partie mesure (10) dans la zone de séparation et une section de liaison complémentaire (25) correspondant à la section de liaison (15) et disposée au niveau de la partie piqûre (20) dans la zone de séparation, et la seconde partie de boîtier formant un capuchon de protection destiné à l'unité de raccordement (14, 14') et
**en ce que** la partie piqûre (20) comporte un élément d'actionnement monté dans la seconde partie de boîtier et accouplé à l'unité de piqûre (21) afin de régler la profondeur de piqûre et/ou de déclencher la fonction de piqûre.

2. Appareil de mesure selon la revendication 1
**caractérisé en ce que**
l'unité de raccordement (14, 14') est conçue pour la connexion à une interface normalisée, par exemple une interface USB, de l'appareil de lecture séparé.

3. Appareil de mesure selon l'une des revendications précédentes
**caractérisé en ce que**
l'unité de raccordement (14, 14') est équipée d'une partie transmission destinée à la transmission de données sans fil, telle que la transmission par infrarouges, la transmission par ultrasons ou la transmission radio.

4. Appareil de mesure selon l'une des revendications précédentes,
**caractérisé en ce qu'**un
dispositif d'affichage comportant une zone d'affichage optique et/ou un logement de moyen de test (11) sont intégrés dans le boîtier de l'appareil, en particulier dans la première partie de boîtier.

5. Appareil de mesure selon l'une des revendications précédentes,
**caractérisé en ce que**
la partie mesure (10) comporte un dispositif de mémorisation dans lequel sont mémorisées les données de mesure acquises au moyen du dispositif d'analyse.

6. Appareil de mesure selon la revendication 5,
**caractérisé en ce que**
la transmission des données de mesure s'effectue automatiquement lors du raccordement de l'appareil de mesure (1) à l'appareil de lecture séparé au moyen de l'unité de raccordement (14, 14').

7. Appareil de mesure selon la revendication 5 ou 6,
**caractérisé en ce que**
des données supplémentaires, en particulier des données à caractère personnel et/ou des données temporelles, sont ou peuvent être stockées dans le dispositif de mémorisation, et
**en ce qu'**un dispositif de gestion de données, au moyen duquel lesdites données complémentaires peuvent être associées aux données de mesure, est présent dans la partie mesure (10).

8. Appareil de mesure selon l'une des revendications précédentes,
**caractérisé en ce que**
l'appareil de mesure (1) est conçu pour le raccordement à un dispositif de mémorisation externe.

9. Appareil de mesure selon l'une des revendications précédentes,
**caractérisé en ce que**
la partie mesure (10) est dotée d'un dispositif de transmission codée des données, en particulier d'un dispositif de chiffrage des données.
